# EUROPEAN PATENT APPLICATION

(11) **EP 4 410 828 A1**
(43) Date of publication of application: **07.08.2024**
(21) Application number: 23154756.3
(22) Date of filing: 02.02.2023
(51) Int. Cl.: C07K 14/81, A61P 1/04, A61P 1/16, A61P 9/00, A61P 11/00, A61P 17/02, A61P 19/02, A61P 29/00, A61P 35/00, A61P 43/00

(54) **OLIGONUCLEOTIDE ENCODING A TISSUE INHIBITOR OF METALLOPROTEASES PROTEIN FOR MEDICAL USE IN MAMMALS**

(71) Applicant: Eberhard-Karls-Universität Tübingen, 72076 Tübingen (DE)
(72) Inventor: Avci-Adali, Meltem, 72138 Kirchentellinsfurt (DE); Golombek, Sonia, 72070 Tübingen (DE)
(74) Representative: Witte, Weller & Partner Patentanwälte mbB

(57) **Abstract**

The present invention relates to oligonucleotides encoding a tissue inhibitor of metalloproteases (TIMP) protein, a method of treatment of diseases and medical conditions associated with insufficient presence of extracellular matrix proteins, use of the oligonucleotide in such treatment, as well as pharmaceutical compositions and medical devices comprising such oligonucleotide.

## Description

The present invention relates to oligonucleotides encoding a tissue inhibitor of metalloproteases (TIMP) protein, a method of treatment of diseases and medical conditions associated with insufficient presence of extracellular matrix proteins, use of the oligonucleotide in such treatment, as well as pharmaceutical compositions and medical devices comprising such oligonucleotide.

### FIELD OF THE INVENTION

The present invention relates to the field of molecular medicine, especially to the protein expression for therapeutic purposes, more specifically to the therapy of cardiovascular diseases such as aneurysms in mammals.

### BACKGROUND

Cardiovascular diseases (CVDs), such as aneurysms, are the leading cause of death worldwide and an economic burden for healthcare systems. Aneurysms are abnormal dilatations of arteries due to local weakening of the vessel wall. They can develop in the aorta (abdominal or thoracic aortic aneurysm), blood vessels supplying the brain (cranial aneurysm), and other parts of the body (peripheral aneurysm).

Aortic aneurysms can lead to dissections and ruptures with massive internal bleeding and an overall mortality of 90 %. Abdominal aortic aneurysms (AAA) are the most common aortic aneurysms. Promoting factors are age, male gender, smoking, genetic predisposition, hypertension, and arteriosclerosis.

The vessel wall of arteries is three-layered. The innermost tunica intima, built from an endothelial cell layer contacting the blood and a basement membrane underneath, is surrounded by the tunica media, containing smooth muscle cells (SMC) and ECM. The ECM contains elastic fibers and collagen fibers, wherein elastic fibers, as elastin, provide elasticity and extensibility, and collagen fibers, as collagen type III, provide stability against tensile stress. The outermost tunica adventitia is built from stable collagen type I and represents the connection to the surrounding tissue.

Discrete reasons for the onset of aneurysm formation are unknown. Aneurysms are associated with a degradation of the ECM, weakening the aortic wall. ECM degradation-products further lead to disease progression by promoting a sterile inflammation leading again to destabilization and destruction of the aortic vessel wall by apoptosis of SMC and upregulation of proteolytic processes. Over time, the blood pressure causes dilatation of the aorta, which may result in aneurysm dissection and/or rupture, followed by massive internal bleeding and thus high mortality.

One of said proteolytic processes is driven by macrophages becoming activated such that matrix metalloproteinases (MMPs) are produced. MMPs are enzymes involved in the physiological turnover of the ECM by degrading its components, such as elastin or collagen. The MMP activity is inhibited by tissue inhibitors of metalloproteinases (TIMP) by covalent binding.

In AAA, especially MMP-9 (or gelatinase B) plasma levels and mRNA expression levels are increased. TIMP-1, the inhibitor of MMP-9 and other MMPs, is a soluble protein secreted into the ECM and is decreased in aortic aneurysms.

Treatments of aneurysms are largely restricted to surgical treatment of late-stage aneurysm patients. Clinically relevant, approved treatments or causative therapies are not available.

Treatments for medical stabilization of the state of the disease rely on regular, systemic administration of antihypertensive drugs. They do not allow restoration of the vessel wall and their use is controversial, since some promote aneurysm formation (Wilmink AB, Vardulaki KA, Hubbard CS, Day NE, Ashton HA, Scott AP, Quick CR. Are antihypertensive drugs associated with abdominal aortic aneurysms? J Vase Surg. 2002 Oct;36(4):751-7). Furthermore, antihypertensive drugs have a high diversity of side effects. Thus, no effective and specific drug therapy to stabilize or heal aneurysms is available.

Therefore, monitoring of aneurysm progression and surgical treatment of patients in late stages, either by open aneurysm repair (OAR) or endovascular aneurysm repair (EVAR), is the only option to mitigate the risk of rupture. However, OAR are associated with a high perioperative mortality and morbidity, while EVAR often needs repeated interventions. Thus, no efficient and safe surgical treatment is available.

Regarding gene-therapies, expression of TIMP-1 by retroviral transduction into rat smooth muscle cells and seeding of those cells onto aneurysm-developing guinea pig aorta xenografts in rats successfully prevented aneurysm dilatation and rupture (Allaire, E., et al., Local overexpression of TIMP-1 prevents aortic aneurysm degeneration and rupture in a rat model. J Clin Invest, 1998. 102(7): p. 1413-20). However, retroviral therapies bear disadvantages such as a mutagenic risk and the permanent integration into the host genome.

WO2013151671A1 discloses polynucleotides for the production of cosmetic proteins, one of which can be TIMP-1.

Thus, it is an object of the present invention to eliminate or at least mitigate the disadvantages of the prior art. Especially, the invention shall provide such an agent which is suitable for improved prophylactic and/or curative therapy of cardiovascular diseases, such as aneurysms and arteriosclerosis.

This object is fully solved by the present invention.

### SUMMARY OF THE INVENTION

In an aspect of the invention, the above-identified disadvantages are overcome by providing an oligonucleotide comprising a nucleotide sequence encoding a 'tissue inhibitor of metalloproteases' (TIMP) protein for use in the treatment of diseases or medical conditions in a mammal.

The TIMP protein family currently has four known members. At least those fall under the scope of the invention, however TIMPs not yet revealed are not excluded. Said four TIMPs are: TIMP-1 (corresponding human Ensembl Gene-ID: ENSG00000102265), TIMP-2 (corresponding human Ensembl Gene-ID: ENSG00000035862), TIMP-3 (corresponding human Ensembl Gene-ID: ENSG00000100234), and TIMP-4 (corresponding human Ensembl Gene-ID: ENSG00000157150).

The oligonucleotide can comprise any sort of natural nucleotide.

Herein, the term "disease" is to be understood as generally in medicine as any kind of unphysiological condition of the respective mammal, which may be caused for example genetically, oncologically, microbially, bacterially, virally, or psychologically. The term "medical condition" is to be understood as referring to any kind of unphysiological condition, which may be caused for example by physical damage of the mammal's body, or aging. However, as generally understood "medical condition" may also refer to diseases resulting from such damage and "disease" may refer to a medical condition. Therefore, the terms "disease" and "medical condition" can be used synonymously herein.

The oligonucleotide is suitable for use in mammals. Preferably such a mammal is a human.

In another aspect of this embodiment, the disease or medical condition is associated with insufficient presence of at least one extracellular matrix protein, wherein the disease or medical condition is preferably selected from the group consisting of: aneurysm, fibrosis, arteriosclerosis, aortic stenosis, pulmonary emphysema, Williams-Beuren-Syndrome, subvalvular congenital aortic stenosis, myocardial infarction, stroke, rheumatoid arthritis, osteoarthritis, and tumor metastasis, more preferably selected from the group consisting of: aortic aneurysm, cranial aneurysm, peripheral aneurysm.

A disease or medical condition associated with "insufficient presence of at least one extracellular matrix protein" is to be understood as any disease or medical condition affecting a mammal, regarding which the mammal may benefit from a stop or retardation of further degradation or an enhancement of the presence of at least one ECM protein of any of its tissues.

Using the oligonucleotide for treatment of diseases associated with insufficient presence of at least one ECM protein advantageously allows to induce a transient TIMP expression in the at least one tissue of a mammal with insufficient presence of at least one ECM protein and thus to reduce the ECM degradation or allow ECM restoration within the respective tissue. This retards the progression and may heal the disease.

In an additional aspect of this embodiment, the disease or medical condition is associated with an unphysiological ratio of MMP-to-TIMP activity.

As described before, MMPs are inhibited by TIMPs. An enhanced MMP activity or a reduced TIMP activity enhances the MMP-to-TIMP ratio and thus the ECM degradation. Thus, an unphysiological MMP-to-TIMP ratio is associated with various diseases, such as CVD, especially aneurysms. The oligonucleotide according to the invention can be used advantageously for enhancing the expression of TIMP proteins in mammals, thus, reducing the ratio of MMP-to-TIMP activity.

The term "unphysiological" has to be understood as a situation in which a mammal may also benefit from a reduction of the ratio of MMP-to-TIMP activity.

In a further aspect of this embodiment, the unphysiological ratio of MMP-to-TIMP activity is associated with an enhanced MMP activity.

In AAA, the MMP activity is enhanced, causing ECM degradation and thus disease progression. By use of the invention, the inventors were advantageously able to reduce the MMP activity in mammal tissue.

In another aspect of this embodiment, the treatment is a prophylactic or therapeutic treatment, wherein preferably the treatment is selected from the group consisting of: prophylaxis of formation or rupture, and healing of aneurysms, said aneurysms are further preferably selected from the group consisting of: aortic aneurysm, cranial aneurysm, peripheral aneurysm.

It is advantageous to conduct a prophylactic treatment with the oligonucleotide, because by reducing the MMP-to-TIMP ratio using the oligonucleotide, the ECM can be protected from MMP activity at a very early stage or before an acute disease develops. However, also a therapeutic treatment of the beginning disease or later stages is possible and advantageous for the health of the patient. Physicians will be able to judge the individual need for treatment onset of the patient, taking advantage of the flexibility the invention provides.

In particular concerning aneurysms, the oligonucleotide has proven efficient for reducing MMP activity, such that a vessel wall weakening, thus formation and/or rupture of an aneurysm can be advantageously prevented or at least retarded. Advantageously, by prophylactic treatment in the early phase, an aneurysm may be healed by restoration of the ECM due to physiological formation while its degradation by MMPs is inhibited.

Prior art aneurysm treatments (OAR and EVAR) require monitoring of disease progression and medical intervention is restricted to late-stage aneurysms. Thus, a prophylactic treatment, made possible by the invention, grossly enhances the possibilities of physicians to act while reducing the risk for the patient.

In an embodiment of the present invention, the oligonucleotide is an oligoribonucleotide, preferably an mRNA.

The therapeutical application of synthetic mRNA has proven promising in the past years due to several advantages over the prior art: First, it can be produced easily by in-vitro-transcription (IVT). Second, it is not integrated into the host genome, and it underlies a physiological decay. Therefore, it persists only transiently in the cell, greatly reducing the mutagenic risk as compared to viral vectors. Also, other side effects, associated with long-term protein overexpression are avoided. Third, it can be more easily delivered into cells due to the smaller size compared to plasmids or viral vectors.

Herein, the terms mRNA and synthetic mRNA are used interchangeably. The term "synthetic" shall make clear that the respective mRNA is artificially produced, preferably by in-vitro transcription (IVT). There may be or may be no structural or chemical differences between a synthetic mRNA and a mRNA.

In another aspect of this embodiment, the oligonucleotide, comprises a 5'-Cap-structure, preferably a synthetic anti-reverse cap analogue, more preferably 3'-O-Me-m7G(5')ppp(5')G, and/or a polyA-tail, preferably consisting of at least approx. 70 adenine nucleotides, further preferably of approx. 120 adenine nucleotides.

Such modifications mimic the natural structure of mammalian mRNA and thus advantageously provide a certain degree of stability to the oligonucleotide such that the oligonucleotide is degraded slower by the respective cell it is delivered to, allowing the oligonucleotide to be translated. A synthetic anti-reverse cap analogue advantageously enhances the efficiency of the IVT reaction and the purity of the IVT reaction product.

The 5'-Cap-structure can be of natural origin. The 5'-Cap-structure can be of synthetic or modified origin, also called 5'-cap-analogue. Therefore, the term "5'-Cap-structure" refers to any natural 5'-Cap-structure naturally used by eukaryotic cells as well as to any synthetic/modified not naturally occurring 5'-Cap-structure or 5'-cap-analogue suitable for replacement of natural 5'-Cap-structures in view of function and cytotoxicity.

Albeit anti-reverse cap analogues are preferred, other 5'-Cap-structures are also suitable. Examples for those are m7G(5')ppp(5')(2'OMeA)pG, m7G(5')ppp(5')(2'OMeA)pU, and m7(3'OMeG)(5')ppp(5')(2'OMeA)pG.

In a further embodiment of the invention, at least one of the nucleotides is an analogue of a naturally occurring nucleotide, wherein the fraction of one type of naturally occurring nucleotide replaced by analogues is at least approx. 5 %, preferably at least approx. 25 %, more preferably approx. 100 %.

Natural, unmodified RNA are difficult to use for therapeutical applications due to its biological instability and immunogenicity, limiting its bioavailability and applicability, respectively. Enzymes degrading RNA are almost ubiquitously present, especially in the extracellular space. Native immune mechanisms involving Toll-like receptors (TLR) recognize single stranded RNA (TLR-7, TLR-8) or double stranded RNA (TLR-3), which subsequently induces an inflammatory immune response.

Advantageously, this embodiment decelerates the degradation of the synthetic mRNA and prohibits the synthetic mRNA to be recognized by the immune system, such that an inflammatory response is avoided.

In this embodiment, a substantial fraction of all nucleotides is an analogue of the respective natural nucleotide. Natural nucleotides are meant to be nucleotides comprising nucleobases naturally occurring in mammalian DNA or RNA. Specifically, those include adenine, guanine, cytosine, thymidine, and uracil. An analogue is to be understood as a chemical structure similar to a natural nucleotide, allowing the cellular translation machinery to translate the nucleotide sequence encoded by the oligonucleotide into an encoded protein while decelerating degradation and/or reducing immunogenicity of the respective oligonucleotide.

The term "substantial fraction" means that not necessarily all of a sort of natural nucleotide need to be replaced by an analogue. At least approx. 5 % of one sort of natural nucleotide are replaced by an analogue, preferably at least approx. 25 %, more preferably approx. 100 %.

In this embodiment, an analogue can be, for example, any of pseudouridine (Ψ), N¹-methylpseudouridine (me¹Ψ), 5-methylcytidine (5mC), phosphorothioate, phosphoramidate, peptide nucleotide, methylphosphonate, 7-deazaguanosine, 2-thiouridine, 5-methyluridine, 5-methyluridine-5'-triphosphate (m5U), 5-iodouridine-5'-triphosphate (15U), 4-thiouridine-5'-triphosphate (S4U), 5-bromouridine-5'-triphosphate (Br5U), 2'-methyl-2'-deoxyuridine-5'-triphosphate (U2'm), 2'-amino-2'-deoxyuridine-5'-triphosphate (U2'NH2), 2'-Azido-2'-deoxyuridine-5'-Triphosphate (U2'N3), 2'-Fluoro-2'-deoxyuridine-5'-triphosphate (U2'F), inosine, 3-methylcytidine, 2-thiocytidine, 2'-methyl-2'-deoxycytidine-5'-triphosphat (C2'm), 2'-amino-2'-deoxycytidin-5'-triphosphate (C2'NH2), 2'-fluoro-2'-deoxycytidine-5'-triphosphate (C2'F), 5-iodocytidin-5'-triphosphate (15U), 5-bromocytidine-5'-triphosphate (Br5C), and 2'-azido-2'-deoxycytidine-5'-triphosphate (C2'N3), 5-azauridine, 2-thio-5-azauridine, 4-thiopseudouridine, 2-thiopseudouridine, 5-hydroxyuridine, 3-methyluridine, 5-carboxymethyl-uridine, 1-carboxymethylpseudouridine, 5-propynyluridine, 1-propynylpseudouridine, 5-taurinomethyluridine, 1-taurinomethylpseudouridine, 5-taurinomethyl-2-thiouridine, 1-taurinomethyl-4-thiouridine, 2-thio-1-methylpseudouridine, 1-methyl-1-deazapseudouridine, 2-thio-1-methyl-1-deazapseudouridine, dihydrouridine, dihydropseudouridine, 2-thiodihydrouridine, 2-thiodihydropseudouridine, 2-methoxyuridine, 2-methoxy-4-thiouridine, 4-methoxypseudouridine, 4-methoxy-2-thiopseudouridine, 5-azacytidine, pseudoisocytidine, N4-acetylcytidine, 5-formylcytidine, N4-methylcytidine, 5-hydroxymethylcytidine, 1-methylpseudoisocytidine, pyrrolocytidine, pyrrolopseudoisocytidine, 2-thio-5-methylcytidine, 4-thiopseudoisocytidine, 4-thio-1-methylpseudoisocytidine, 4-thio-1-methyl-1-deazapseudoisocytidine, 1-methyl-1-deazapseudoisocytidine, zebularine, 5-azazebularine, 5-methylzebularine, 5-aza-2-thiozebularine, 2-thiozebularine, 2-methoxycytidine, 2-methoxy-5-methylcytidine, 4-methoxypseudoisocytidine, 4-methoxy-1-methylpseudoisocytidine, 7-deazaadenine, 7-deaza-8-azaadenine, 1-methyladenosine, N6-methyladenosine, N6-isopentenyladenosine, N6-(cis-hydroxyisopentenyl)-adenosine, 2-methylthio-N6-(cis-hydroxyisopentenyl)-adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthioadenine, 2-methoxyadenine, 1-methylinosine, 7-deaza-8-azaguanosine, 6-thioguanosine, 6-thio-7-deazaguanosine, 6-thio-7-deaza-8-azaguanosine, 7-methylguanosine, 6-thio-7-methylguanosine, 7-methylinosine, 6-methoxyguanosine, 1-methylguanosine, N2-methylguanosine, N2,N2-dimethylguanosine, 8-oxo-guanosine, 7-methyl-8-oxoguanosine, l-methyl-6-thioguanosine, or N2-methyl-6-thioguanosine or combinations thereof.

In an aspect of this embodiment, the natural occurring nucleotides uridine and/or uridine are replaced by nucleotide analogues, preferably by a nucleotide analogue or a combination of analogues selected from the group consisting of: a combination of pseudouridine for replacement of uridine and 5-methylcytidine for replacement of cytidine, a combination of N¹-methylpseudouridine for replacement of uridine and 5-methylcytidine for replacement of cytidine, and N¹-methylpseudouridine for replacement of uridine, most preferably N¹-methylpseudouridine for replacement of uridine.

Replacement of said nucleotides by said analogues have turned out to be highly suitable for reduction of immunogenicity of the oligonucleotide, thus allowing cellular survival of the treatment, as well as allowing TIMP expression.

Surprisingly, replacing uridine by N¹-methylpseudouridine without replacing cytidine, lead to the most efficient and highest TIMP expression. Therefore, it is highly preferred in this embodiment, to solely replace uridine by N¹-methylpseudouridine.

Also in this embodiment, the fraction of one type of naturally occurring nucleotide replaced by analogues is at least approx. 5 %, preferably at least approx. 25 %, more preferably approx. 100 %.

In another embodiment of the present invention, the TIMP protein is a TIMP-1 protein. The TIMP-1 protein is an inhibitor for a wide variety of MMP and thus advantageously allows efficient reduction of MMP activity.

In another embodiment of the present invention, the TIMP-1 protein comprises the amino acid sequence of SEQ ID NO: 1.

Advantageously, the use of this human TIMP-1 amino acid sequence allows to correctly express TIMP-1 in a human cell such that the oligonucleotide can be used in medical applications targeting humans without a risk of immune reactions, cellular toxicity, or dysfunctional protein.

In another embodiment of the present invention, the oligonucleotide comprises the nucleotide sequence of SEQ ID NO: 2.

Advantageously, the use of this human TIMP-1 nucleotide sequence allows to translate and thus express TIMP-1 in a human cell efficiently, such that the oligonucleotide can be used in medical applications targeting humans. The sequence shows low immunogenicity and a high expression efficiency.

In another embodiment, the oligonucleotide is complexed by polymers or lipids, preferably contained within a lipid nanoparticle, or contained within a liposome. This advantageously further protects the oligonucleotide from degradation, reduces its immunogenicity, and facilitates its delivery, by improving its entering into the cell.

In another aspect, the present invention relates to a pharmaceutical composition, comprising the oligonucleotide for use of any of the preceding claims and a pharmaceutically acceptable carrier.

A suitable carrier for the specific treatment will be clear to the skilled person. Additional disclosure of pharmaceutically acceptable carriers of synthetic mRNA can be found for example in Ouranidis A, et al., mRNA Therapeutic Modalities Design, Formulation and Manufacturing under Pharma 4.0 Principles. Biomedicines, 2021. Dec 27;10(1):50. The content thereof is herein incorporated by reference.

In one embodiment, the pharmaceutical composition is configured for local administration by injection into or external application onto the tissue of the mammal, wherein preferably the pharmaceutical composition is present in a formulation or delivery form selected from the group consisting of: a fluid, an implant, a balloon catheter, a vascular prosthesis, a microneedle, a patch, and a stent.

The term "fluid" is to be understood as material continuously deforming when experiencing shear forces. A fluid may have the state of a gas, aerosol, or a liquid. A local administration is advantageous since it allows to specifically induce TIMP expression at the site of disease or medical condition without unnecessarily affecting other regions of the body not requiring ECM protection or restoration, which may cause harm to the respective mammal.

Any type of external application further is advantageous in that it is less invasive and thus less stressful than other modes of application.

The above-mentioned formulation or delivery form of a fluid is advantageous because fluids allow the application of the pharmaceutical composition by injection, inhalation or the like into virtually any region of the body, no matter the size of the region. In this context the delivery form of a microneedle or a patch allows a low- or non-invasive application, respectively.

The above-mentioned formulation or delivery form of an implant is advantageous because it allows a repeated or constant, possibly automated, local application of the oligonucleotide into the desired tissue without a need for prolonged or repeated medical intervention, except placement and, possibly, removal of the implant.

The formulation or delivery form of a balloon catheter, a vascular prosthesis, or a stent advantageously allow to apply the pharmaceutical composition to blood vessels and are widely used and approved for treatment of aneurysms.

In one embodiment, the pharmaceutical composition is configured for systemic administration, wherein preferably the pharmaceutical composition is present in a formulation or delivery form selected from the group consisting of: a fluid or an implant.

The above-mentioned formulation or delivery form of a fluid is advantageous because fluids allow the application of the pharmaceutical composition by injection into virtually any region of the body, no matter the size of the region.

The above-mentioned formulation or delivery form of an implant is advantageous because it allows a repeated or constant, possibly automated, local application of the oligonucleotide into the desired tissue without a need for prolonged or repeated medical intervention, except placement and, possibly, removal of the implant.

In another embodiment, the pharmaceutical composition contains or is administered in combination with at least one antihypertensive drug. Especially in treatment of acute and late-stage aneurysms, this may be advantageous for preventing the imminent danger of a rupture or dissection.

Another aspect of the present invention is a medical device, characterized in that the medical device comprises, includes, or is coated with the oligonucleotide.

In one embodiment, the medical device is configured for permanent or transient presence in the body of the mammal, wherein preferably the medical device is selected from the group consisting of: an implant, a balloon catheter, a vascular prosthesis, a microneedle, a patch, and a stent. This advantageously allows an adjustment of the duration and spatial extension of the treatment to the individual needs of the patient by the physician.

The features, characteristics, and advantages mentioned for the oligonucleotide according to the invention likewise apply to the pharmaceutical composition and the medical device according to the invention.

Another aspect of the invention is a method of treatment of diseases or medical conditions associated with insufficient presence of at least one extracellular matrix protein. The method of treatment comprises the application of the oligonucleotide and/or the pharmaceutical composition and/or the medical device according to the invention to a living mammal suspected of being affected by said medical condition. The application can be accomplished locally or systemically.

In one embodiment, the method may comprise to repeat the application of the oligonucleotide and/or pharmaceutical composition and/or medical device at least once. A repeated application allows to reduce ECM degradation or restore ECM within the respective at least one tissue for a prolonged period or repeatedly, which may be necessary since an oligonucleotide will transiently and non-permanently cause TIMP expression.

As a convention, it is understood that all nucleotide sequences of the present disclosure are given in terms of DNA sequences. However, the sequences also include RNA sequences. The RNA sequence corresponding to a specified DNA sequence results from the fact that all thymines (T, t) are replaced by uracils (U, u). Such an exchange takes place in particular when reference is made to mRNA molecules, but a DNA sequence is explicitly specified. This means, for example, that the nucleotide sequence SEQ ID NO: 4: TTGGACCCTC GTACAGAAGC TAATACG specified in the form of DNA comprises the following RNA nucleotide sequence: UUGGACCCUC GUACAGAAGC UAAUACG. The same applies to the nucleotide sequences of the remaining DNA/RNA molecules according to the invention.

It is understood that the features mentioned above and those yet to be explained below can be used not only in the particular combination given, but also in other combinations or in isolation, without departing from the scope of the present invention.

The invention is now described and explained in further detail by referring to the following non-limiting examples and drawings.

### BRIEF DESCRIPTION OF THE FIGURES

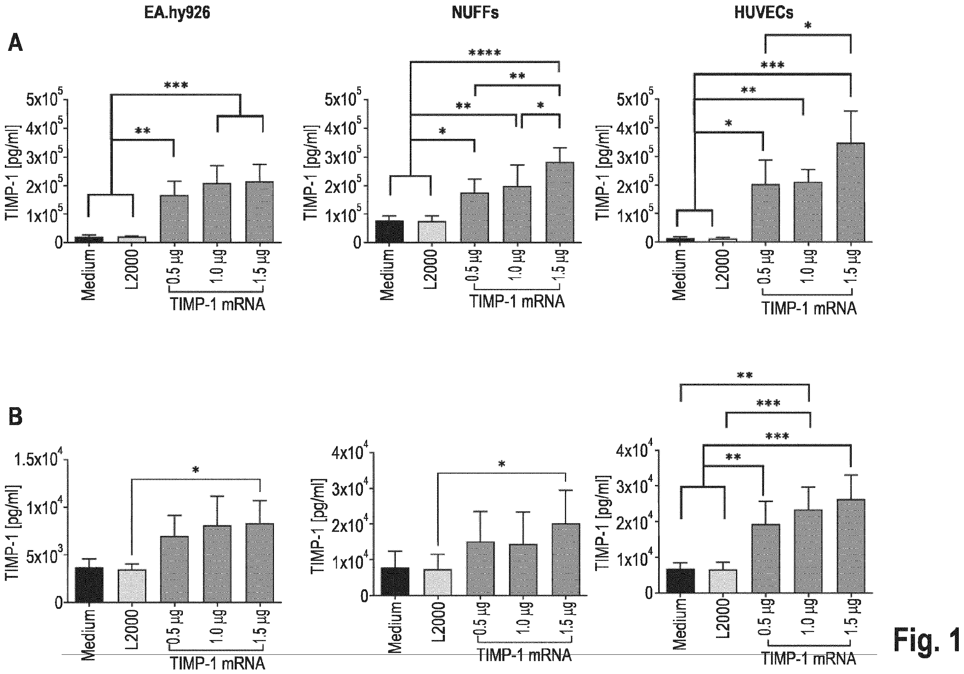

Fig. 1: Analysis of TIMP-1 protein expression after transfection of different cell lines with synthetic TIMP-1 mRNA. 3×10⁵ EA.hy926 cells, NUFFs, or HUVECs were transfected with 0.5, 1.0, 1.5 µg TIMP-1 mRNA complexed 1:1 with L2000. After 4 h incubation at 37 °C and 5 % CO₂, the transfection complexes were replaced with the appropriate corresponding cell culture medium. After an additional incubation for 24 h at 37 °C and 5 % CO₂, TIMP-1 protein concentration in (A) supernatants and (B) cell lysates was determined by human TIMP-1 specific ELISA. Cells treated with medium or medium containing L2000 served as controls. Results are shown as mean + SD (n=3). Statistical differences were determined using one-way ANOVA followed by Tukey's multiple comparisons test. (*p < 0.05, **p < 0.01, ***p < 0.001, ****p < 0.0001).

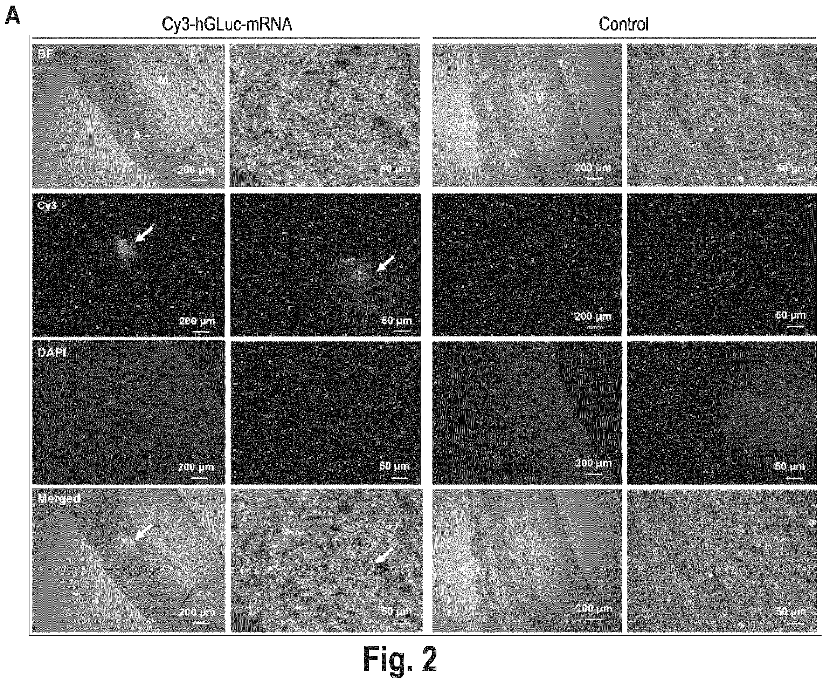

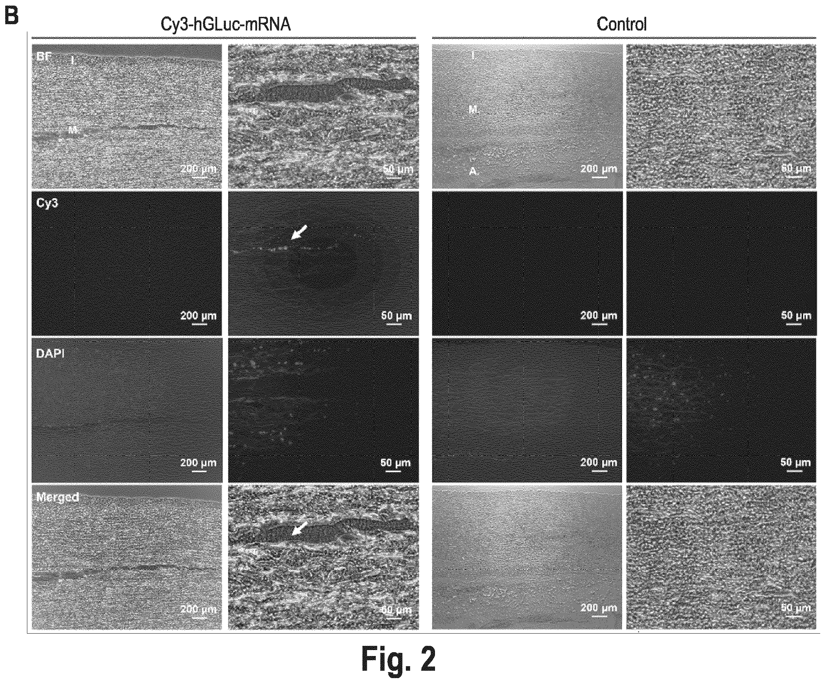

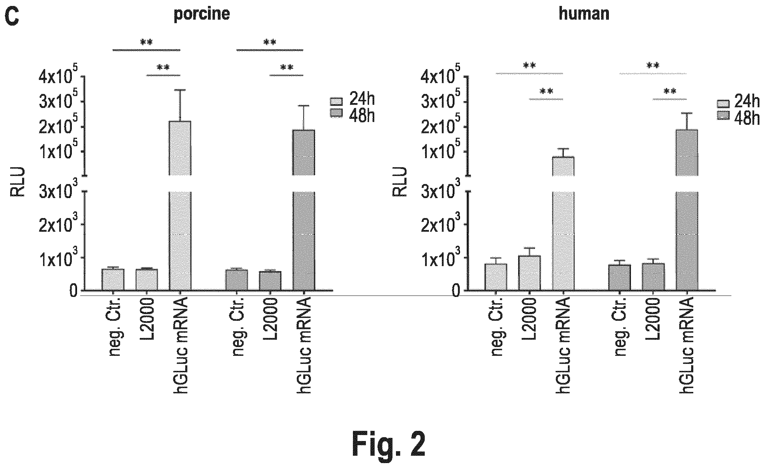

Fig. 2: Application of synthetic hGLuc mRNA into ex vivo porcine and human aortic vessel. Injection of 1 µg of Cy3-labeled hGLuc mRNA complexed with 1 µl L2000 in 50 µl Opti-MEM into porcine (A) and human (B) aortic vessel from the intraluminal site. After microinjection, samples were fixed in 4% PFA and paraffin sections were prepared (2.5 µm). Cell nuclei were stained with DAPI. White arrows indicate the Cy3-labeled hGLuc mRNA in the tunica media and the adventitia. DAPI (blue), Cy3-labeled hGLuc mRNA (red). I.: Tunica intima, M.: Tunica media, A.: Tunica adventitia. (C) Detection of luciferase activity after ex vivo microinjection of 1 µg synthetic hGLuc mRNA complexed with 1 µl L2000 in the porcine aorta (left) and human aorta (right). Cell supernatants were analyzed by luciferase assay after incubation at 37 °C and 5 % CO₂ for 24 and 48 h, respectively, during which the medium was not changed. Aortic vessels injected with medium or medium containing 1 µl L2000 were used as control. For statistical analysis, a two-way ANOVA with subsequent Bonferroni correction was performed (**p ≤ 0.01). The results are shown as means + SEM (pig n=4, human n=5).

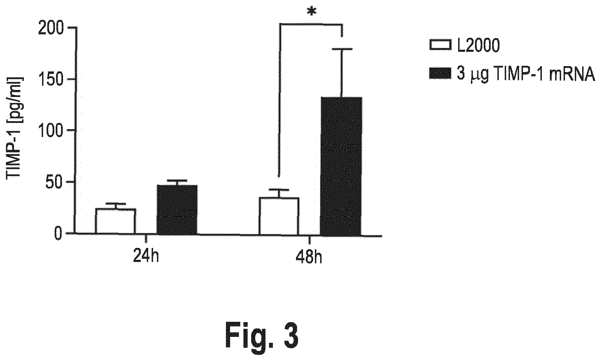

Fig. 3: Detection of TIMP-1 levels in the supernatant of porcine aorta after the microinjection of 3 µg TIMP-1 mRNA. Injection of 3 µg of TIMP-1 mRNA complexed with L2000 in 50 µl Opti-MEM into porcine aortic vessel. TIMP-1 levels in the supernatant were detected after 24 and 48 h post injection using TIMP ELISA. The results are shown as means + SEM (n=3). Statistical differences were determined using two-way ANOVA followed by Tukey's multiple comparisons test. (*p < 0.05).

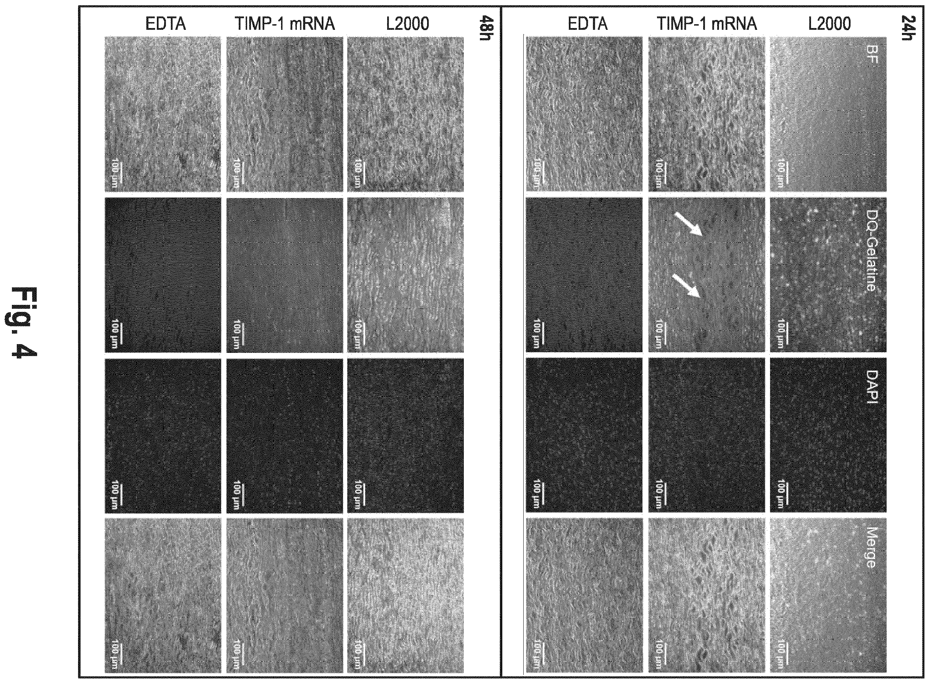

Fig. 4: Analysis of MMP activity using in situ zymography in porcine aorta after microinjection of porcine TIMP-1 mRNA and incubation for 24 and 48 h. 5 µg of TIMP-1 mRNA complexed 1:1 with L2000 in 50µl Opti-MEM were injected ex vivo from the side of the intima into porcine aorta. Tissues were incubated for 24 and 48 h at 37 °C and 5 % CO₂. Injection of L2000 only in Opti-MEM served as a control. Post-incubation, tissues were fixed and 5 µm paraffin sections were prepared. MMP-9 activity was visualized by using DQ-gelatin based in situ zymography. To verify enzymatic gelatin cleavage by MMPs, control sections were inhibited with EDTA before the addition of DQ substrate. Cell nuclei were stained using DAPI containing mounting medium. DQ-gelatin: green, Nuclei: blue. 20x magnification; (n=3).

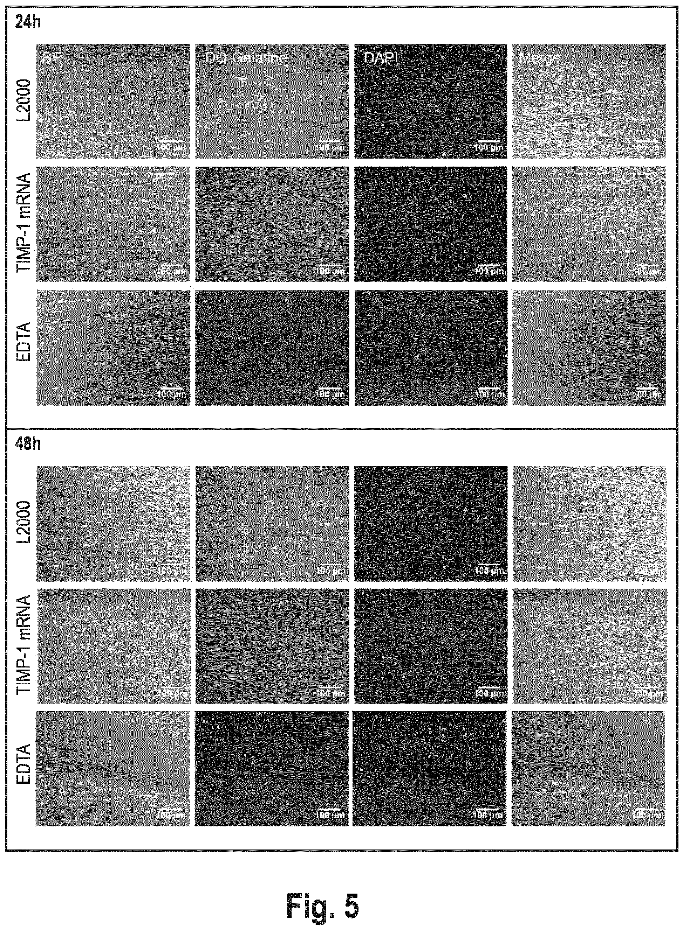

Fig. 5: Analysis of MMP activity using in situ zymography in human aorta after microinjection of human TIMP-1 mRNA and incubation for 24 and 48 h. 5 µg of TIMP-1 mRNA complexed 1:1 with L2000 in 50 µl Opti-MEM were injected ex vivo from the side of the intima into human aorta. Tissues were incubated for 24 and 48 h at 37 °C and 5 % CO₂. Injection of L2000 only in Opti-MEM served as a control. Post-incubation tissues were fixed, 5 µm paraffin sections were prepared and MMP-9 activity was visualized by using DQ-gelatin based in situ zymography. To verify enzymatic gelatin cleavage by MMPs, control sections were inhibited with EDTA before the addition of DQ substrate. Cell nuclei were stained using DAPI containing mounting medium. DQ-gelatin: green, Nuclei: blue. 20x magnification; (n=3).

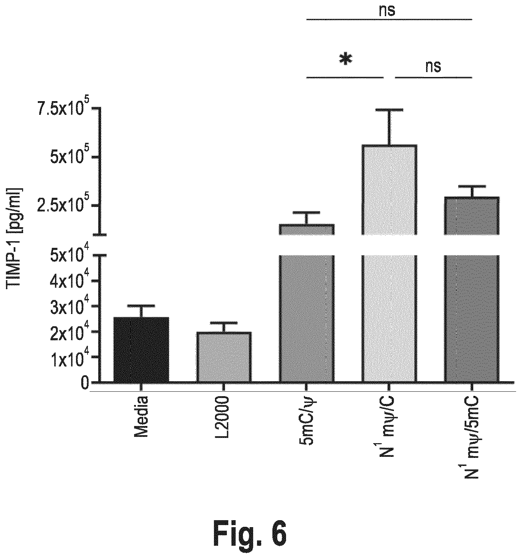

Fig. 6: Impact of nucleotide modification on synthetic TIMP-1 mRNA expression efficiency in EA.hy926 cells. 3×10⁵ EA.hy926 cells were seeded and transfected with 1.5 µg of TIMP-1 mRNA complexed with 1.5 µl of Lipofectamine2000 in OptiMEM for 4 h at 37 °C and 5 % CO₂. Thereafter, the transfection complexes were replaced by cell culture medium and the cells were incubated at 37 °C and 5 % CO₂. Cells treated with OptiMEM or OptiMEM plus L2000 served as controls. After 24 h, TIMP-1 concentration was determined in collected supernatants via human TIMP-1 specific ELISA. Results are shown as mean + SEM (n = 3). Statistical differences between mRNA-treated groups were determined using one-way ANOVA following Bonferroni's multiple comparisons test (*p < 0.05, ns: non-significant).

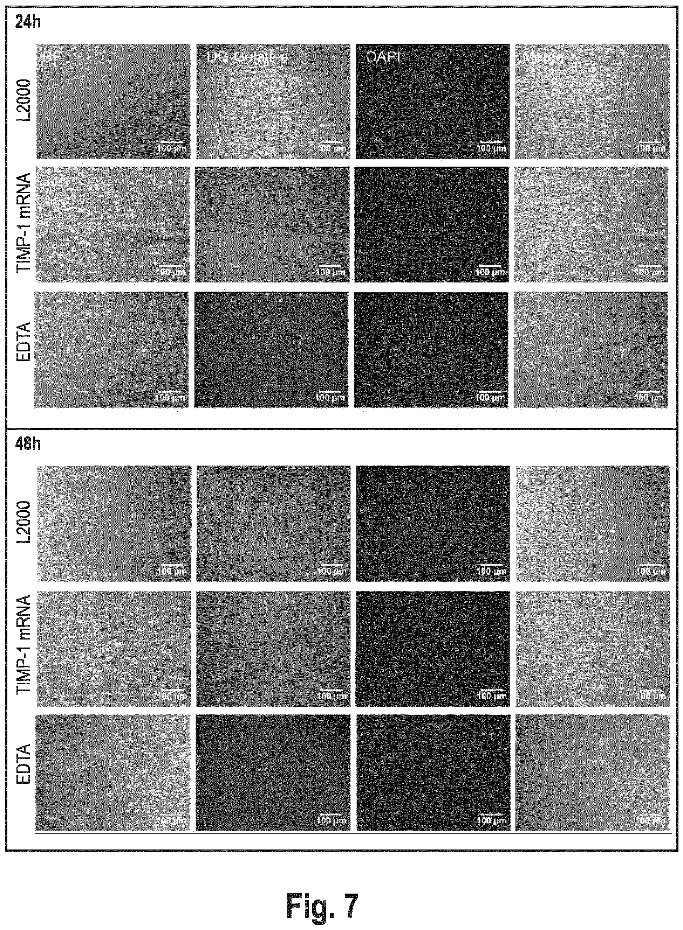

Fig. 7: Analysis of MMP activity using in situ zymography in porcine aorta after microinjection of human TIMP-1 mRNA from the side of the adventitia. 5 µg TIMP-1 mRNA complexed 1:1 with L2000 in 50µl Opti-MEM were injected from the side of the adventitia and incubated for 24 h and 48 h at 37 °C and 5 % CO₂. L2000 in Opti-MEM served as a control. Post incubation tissues were fixed, 5 µm paraffin sections were prepared and MMP-9 activity was visualized by using DQ-gelatin based in situ zymography. To verify enzymatic gelatin cleavage by MMPs, control sections were inhibited with EDTA before addition of substrate. Cell nuclei were stained using DAPI containing mounting medium. DQ-gelatin: green, Nuclei: blue. 20x magnification; (n=3).

### EXAMPLES

### 1. Materials and methods

### 1.1 Cultivation of cells

EA.hy926 cells (ATCC) and human fibroblasts (NUFFs, newborn foreskin fibroblasts, AMS Biotechnology (Europe) Ltd., Abingdon, United Kingdom) were cultivated in Dulbecco's modified eagle medium (DMEM) with high glucose and L-glutamine with 10 % heat-inactivated fetal bovine serum (FBS) at 37 °C and 5 % CO₂. Cells were washed once with Dulbecco's phosphate-buffered saline (DPBS) and detached with 0.05 % trypsin-EDTA. All cell culture reagents were obtained from Thermo Fisher Scientific (Waltham, MA, USA).

Human umbilical vein endothelial cells (HUVECs, (PromoCell, Heidelberg, Germany)) were cultivated in VascuLife EnGS endothelial cell culture medium (without hydrocortisone) (Lifeline Cell Technology, Frederick, MD, USA) at 37 °C and 5 % CO₂. Cells were washed once with DPBS and detached using 0.04 % trypsin/0.03 % EDTA and trypsin neutralizing solution (TNS) 0.05 % in 0.1 % BSA (both Promocell, Heidelberg, Germany). Cell culture flasks were coated with 0.1 % gelatin (Fluka, Morris-town, USA) in PBS for 15 min at room temperature (RT) before seeding the cells. The medium was changed every 3 - 4 days and cells were passaged upon reaching a confluency of 80-90 %.

### 1.2 mRNA synthesis

Synthetic mRNA was produced by in vitro transcription (IVT) as previously described (Avci-Adali, M., et al., In vitro synthesis of modified mRNA for induction of protein expression in human cells. JoVE (Journal of Visualized Experiments), 2014(93): p. e51943). Plasmids containing either secretable humanized Gaussian luciferase (hGLuc, SEQ ID NO: 3) or human TIMP-1 (hTIMP-1, SEQ ID NO: 2) encoding sequences were ordered from Eurofins Genetics (Ebersberg, Germany). These plasmids were used to generate the DNA templates containing the coding sequence of hGLuc or hTIMP-1 by PCR. Therefore, 50-100 ng of plasmid and the HotStar HiFidelity Polymerase Kit (QIAGEN, Hilden, Germany) were used according to the manufacturer's instructions. Forward (SEQ ID NO: 4) and reverse primer (SEQ ID NO: 5) were purchased from ELLA Biotech (Martinsried, Germany). PCR was performed using the following PCR cycling conditions: 94 °C for 3 min, 30 cycles of denaturation to generate single strands at 94 °C for 45 s, hybridization of primers at 60°C for 1 min, elongation at 72 °C for 1 min, and final elongation at 72 °C for 5 min. The amplified PCR products were purified using the QI-Aquick PCR purification kit (QIAGEN, Hilden, Germany) according to the manufacturer's instructions. The concentration and purity of the DNA were analyzed photometrically, and the size of the amplified DNA fragments was controlled by 1 % agarose gel electrophoresis.

IVT was performed using T7 MEGAscript Kit (Life Technologies, Darmstadt, Germany) according to the manufacturer's instructions. Therefore, 1.5 µg of the hGLuc or hTIMP-1 template DNA was used. During the IVT, 2.5 mM 3'-O-Mem7G(5')ppp(5')G RNA cap structure analog (ARCA, New England Biolabs, Frankfurt am Main, Germany) and nucleotides (1.875 mM GTP, 7.5 mM ATP, 7.5 mM 5-methyl-CTP (5mC), 7.5 mM Ψ) . were incorporated. This mRNA modification was named Ψ/5mC. To analyze the impact of nucleotide-modification on TIMP-1 protein expression, TIMP-1 mRNA was also produced with 7.5 mM N¹-methylpseudouridine (me¹Ψ) instead of Ψ and 7.5 mM 5mC (named me¹Ψ/5mC) or 7.5 mM CTP (named me¹Ψ/C). ATP, GTP, and CTP were used from MEGAscript T7 Kit and the others from TriLink (BioTechnologies, San Diego, USA). To each IVT reaction, 40 U RiboLock RNase inhibitor (Thermo Scientific, Waltham, MA, USA) was added and the IVT reaction was incubated for 4 h at 37°C. For the removal of the DNA template, 1 µl of TurboDNase was added and incubated for a further 15 min before performing the mRNA purification with the RNeasy Mini Kit (Qiagen, Hilden, Germany) according to the manufacturer's instructions. Subsequently, mRNA was dephosphorylated at 37 °C for 30 min with 15 U Antarctic phosphatase (New England Biolabs, Frankfurt am Main, Germany) and purified as above described. The concentration and purity of mRNAs were analyzed photometrically, and the size of the mRNAs was confirmed by 1 % agarose gel electrophoresis and staining with 1x GelRed (Biotium, Fremont, CA, USA) in 1x Tris-borate-EDTA (TBE) buffer for 1 h at RT.

### 1.3 Cy3-labeling of hGLuc mRNA

To evaluate the localization of the mRNA in the blood vessel walls, hGLuc mRNA was labeled with the fluorophore Cy3. Therefore, Cy3 was linked to the mRNA using a copper-free click chemistry reaction. During IVT, 5-azido-C₃-UTP (Jena Bioscience, Jena, Germany) molecules were integrated into the mRNA and subsequently labeled with Cy3. For this purpose, 25 % 5-azido-C₃-UTP (1.875 mM) and 75 % Ψ (5.625 mM) were used for IVT as described in our previous study [31]. The azido mRNA was purified and coupled with DBCO-sulfo-Cy3 (Jena Bioscience) in a 5:1 ratio in a total volume of 40 µl nuclease-free water for 1 h at 37 °C. The labeled mRNA was purified using the RNeasy Mini Kit (Qiagen, Hilden, Germany) and evaluated by 1 % agarose gel electrophoresis.

### 1.4 Transfection of cells with TIMP-1 mRNA

3×10⁵ HUVECs, NUFFs, or EA.hy926 cells were seeded per well of a 6-well plate and cultivated overnight at 37 °C and 5 % CO₂ in the respective cell culture medium. Before the seeding of HUVECs, cell culture plates were coated with 0.1 % gelatin in PBS for 15 min. Cells were transfected with 0.5, 1 µg, or 1.5 µg synthetic mRNA complexed with Lipofectamine 2000 (L2000) in a 1:1 ratio in 1 ml Opti-MEM (except for 0.5 µg mRNA, where 1 µl L2000 was used). Opti-MEM alone or Opti-MEM with 1.5 µl Lipofectamine 2000 was used as controls. For the analysis of modified nucleotides on the TIMP-1 mRNA expression efficiency, 1.5 µg of each modified TIMP-1 mRNA variant was complexed with 1.5 µl L2000 and transfected in EA.hy926 cells. Transfection mixtures were incubated for 20 min at RT. Before adding transfection mixtures, cells were washed once with DPBS, and cells were incubated for 4 h at 37 °C and 5 % CO₂ with the transfection complexes. Then, the transfection mixtures were replaced with 2 ml of cell culture medium, and the cells were incubated for another 24 h at 37 °C and 5 % CO₂. Cell culture supernatants were collected, snap-frozen in liquid nitrogen, and stored at -80°C until performing TIMP-1 protein-specific ELISA. Furthermore, cells were lysed to detect intracellular TIMP-1 protein amounts by ELISA.

### 1.5 Lysis of cells after the TIMP-1 mRNA transfection

Cells were washed 2x with 1 ml cold DPBS, overlaid with 300 µl cold 1x RIPA buffer with 1:100 Halt Protease Inhibitor Cocktail (both from Thermo Fisher Scientific Waltham, MA, USA), and incubated on ice for 5 min. The cells were then lysed by repeated pipetting, transferred to a reaction tube, and treated in an ultrasonic bath for 3 × 10 s with a 10 s pause in between. Subsequently, cell lysates were centrifuged at 13,000 rpm, 4 °C for 25 min. The supernatant was collected, snap-frozen in liquid nitrogen, and stored at -80 °C until performing TIMP-1 protein-specific ELISA.

### 1.6 Application of synthetic mRNA into blood vessel walls

### 1.6.1 Tissue preparation

Porcine and human aortic tissues were transferred to 0.9 % NaCl solution immediately after collection and used for microinjections on the same day. Before microinjection, the tissue was cut into 0.5 × 0.5 cm pieces and incubated for 30 min in an antibiotic solution composed of 250 mg/mL gentamicin (Sigma-Aldrich, St. Louis, USA) and 1.25 mg/mL amphotericin B (PromoCell, Heidelberg, Germany) in DMEM followed by washing with DPBS (both from Thermo Fisher Scientific (Waltham, MA, USA)).

### 1.6.2 Injection of synthetic hGLuc or TIMP-1 mRNA

To analyze synthetic mRNA-mediated protein expression in the aortic vessel wall, synthetic mRNA was delivered ex vivo to porcine and human aortic vessel walls by microinjection from the side of the intima using MicronJet600 hollow microneedles from NanoPass Technologies (Nes Ziona, Israel). Therefore, 1 µg of hGLuc mRNA or 3 or 5 µg TIMP-1 mRNA was complexed in a 1:1 ratio (µg:µl) with Lipofectamine2000 (L2000) and incubated in a total volume of 50 µl Opti-MEM for 20 min at RT. The prepared mRNA solutions were drawn into a 1 ml (Luer-Lock) syringe using a cannula, which was replaced by the microinjection needle for the injection from the intraluminal side.

After injection, samples were incubated for 5 min at RT, washed once in DPBS, and incubated at 37 °C and 5 % CO₂ in 1 ml EC medium in a 12-well plate for 24 to 48 h to analyze the luciferase activity, TIMP-1 amount by ELISA and the activity of exogenously produced TIMP-1 in the vessel wall using in situ zymography. Microinjection of 50 µl Opti-MEM and the corresponding amount L2000 into tissue served as controls. For the in-situ zymography, tissue samples were incubated for 48 and 72 h at 4 °C in 10x zinc fixative (Formalin free, BD Pharmingen, Becton, Dickinson and Company, Franklin Lakes, USA).

To evaluate the localization of the mRNA after the microinjection into the blood vessel wall, 1 µg Cy3 labeled hGLuc-mRNA was complexed with 1 µl L2000 in 50 µl Opti-MEM for 20 min at RT. Tissue treated with Opti-MEM and the corresponding amount L2000 served as control. After the microinjection, tissues were washed 1x in DPBS and fixed overnight in 4% paraformaldehyde (PFA) at 4 °C for histological analyses.

### 1.6.3 Detection of luciferase activity

After the delivery of synthetic hGLuc mRNA into the aortic vessel wall, the expression of hGLuc in tissue supernatants was determined using a luciferase assay. Therefore, 40 µl of each supernatant was transferred in triplicates in a 96-well plate (Nunc Maxisorp, Thermo Fisher Scientific (Waltham, MA, USA)) and 100 µl of 20 µg/ml coelenterazine (Carl Roth, Karlsruhe, Germany) in DPBS (w/o Ca²⁺/Mg²⁺) was injected automatically into each well, and luminescence was detected in Relative Light Units (RLU) using the microplate reader Mithras LB 940 (Berthold Technologies, Bad Wildbad, Germany).

### 1.7 Histological analysis

### 1.7.1 Detection of microinjected mRNA in the aortic vessel wall

After fixation, the samples were washed twice in 2 ml DPBS, transferred to embedding cassettes, and coated with 100 % ethanol. Subsequently, automated dehydration and embedding in paraffin were performed by the Institute of Pathology at the University Hospital of Tübingen. Subsequently, tissue sections with a thickness of 2.5 µm were prepared. Afterwards, the sections were deparaffinized 4 × for 5 min in xylene rehydrated stepwise in an ethanol series: 2x 99 % for 1 min, 2x 96 % for 1 min, 2x 70 % for 1 min, washed twice in nuclease-free water, and then boiled for 2 min in TBE buffer (pH 9). The slides were cooled under running water, washed three times with DPBS, and in the case of Cy3 mRNA injection, embedded in Fluoroshield Mounting Medium with DAPI (Vector Laboratories, Burlingame, CA, USA). Xylene and ethanol were both provided by AnalaR NORMAPUR (VWR, Darmstadt, Germany). To detect Cy3-labeled mRNA, fluorescence images were taken using the Axiovert135 microscope (Carl Zeiss, Oberkochen, Germany) and analyzed with AxioVision Rel 4.8 software.

### 1.7.2 In situ zymography

After fixation, the tissue was transferred to embedding cassettes, dehydrated, and embedded using a tissue embedding device and an automated tissue processor at the Institute of Pathology at the University Hospital of Tübingen. The tissue was then poured into paraffin blocks and 5 µm thick sections were prepared using the Microtome Microm HM 355S (Thermo Fisher Scientific (Waltham, MA, USA)) and mounted on SuperFrost microscope slides (R. Langenbrinck, Emmendingen, Germany). Tissue sections were deparaffinized in xylene (100 % xylene, mixture of isomers, AnalaR NORMAPUR, VWR, Darmstadt, Germany) two times for 1 min and rehydrated using descending ethanol (AnalaR NORMAPUR, VWR, Darmstadt, Germany) series (100 %, 80 %, 70 %, 60 %). The slides were washed in double-distilled water (ddH2O) and stained in a humidity chamber using EnzChek Gelatinase/Collagenase Assay Kit (Thermo Fisher Scientific (Waltham, MA, USA)) by adding 250 µl substrate solution consisting of 1 mg/ml of the fluorescence-labeled DQ-gelatin diluted 1:50 in reaction buffer ((150 mM NaCl (NORMAPUR^{®}, VWR International, LLC., Radnor, USA), 5 mM CaCl₂, 50 mM Tris-HCl, 0.2 mM sodium azide (all from Sigma-Aldrich, Darmstadt, Germany); pH 7.6)) to each section and incubation at 37 °C for 2 h. As a control, tissue sections were treated for 1 h with 20 mM EDTA to inhibit enzymatic metalloproteinase activity. The slides were then incubated at 37 °C for 2 h with the substrate solution. Afterwards, the sections were washed 3x for 1 min in ddH2O and fixed in 250 µl 4% PFA for 10 min in the dark, followed by 2x washing for 5 min in DPBS. Finally, the tissue sections were covered with Vectashield mounting medium containing DAPI (Vector Laboratories, Newark, USA) to stain the cell nuclei. Images were taken using the Axiovert135 fluorescence microscope (Carl Zeiss, Oberkochen) and analyzed using AxioVision Rel 4.8 program software.

### 1.8 Human TIMP-1 ELISA

TIMP-1 amount was detected in the cell lysates and cell culture supernatants after the TIMP-1 mRNA transfection and the microinjection of mRNA into aortic tissues. The amount of TIMP-1 was detected using human TIMP-1 DuoSet ELISA (R&D Systems, Minneapolis, MN, USA) according to the manufacturer's instructions. Cell supernatants and lysates were diluted (cell supernatants: 1:150-1:250, cell lysates 1:50) in 1 % BSA in DPBS before ELISA. Supernatants of aortic tissue vessels cultivations were not diluted. The absorbance of the samples was measured using a microplate reader (Eon Synergy 2, BioTek Instruments) at 450 nm with a correction wavelength of 540 nm.

### 1.9 Statistical analysis

The data are shown as mean + standard deviation (SD) or standard error of the mean (SEM). One- or two-way analysis of variance (ANOVA) was performed followed by Tukey's multiple comparisons test or Bonferroni's multiple comparisons test. All the analyses were performed using GraphPad Prism version 9.3.1. Differences of p < 0.05 were considered statistically significant.

### 2. Results

### 2.1 Transfection of cells with synthetic TIMP-1 mRNA

To analyze TIMP-1 protein production, 3x 10⁵ EA.hy926 cells, NUFFs, or HUVECs were transfected with 0.5, 1.0, or 1.5 µg synthetic TIMP-1 mRNA. Using ELISA, significantly increased TIMP-1 levels were detected in the supernatants of all cell types 24 h after the TIMP-1 mRNA transfection (Figure 1A). Except for EA.hy926 cells, increasing the TIMP-1 mRNA concentration to 1.5 µg resulted in a significant increase in TIMP-1 protein expression. Higher levels of TIMP-1 were detected in the supernatants of the cells than in the cell lysates, indicating the secretion of TIMP-1 protein into the extracellular space after translation. In EA.hy926 cells and NUFFs, only transfection of 1.5 µg TIMP-1 mRNA resulted in significantly higher TIMP-1 levels in cells compared with cells transfected with transfection reagent (L2000) alone (Figure 1B). In HUVECs, transfection of 0.5 µg TIMP-1 mRNA already resulted in significantly increased TIMP-1 levels in cells.

### 2.2 Delivery of synthetic mRNA into the aortic vessel wall by microinjection

To investigate the delivery of synthetic mRNA into the blood vessel wall, 1 µg of Cy3-labeled hGLuc mRNA was injected ex vivo using hollow microneedles from the intraluminal site into porcine and human aortic vessels. In paraffin cross-sections, the localization of Cy3-labeled hGLuc mRNA was analyzed by fluorescence microscopy. In these histologic sections, the individual compartments of the vessels, intima, tunica media, and tunica adventitia, were readily recognizable. The cell nuclei were stained with DAPI (blue). Cy3-labeled hGLuc mRNA (red) was detected mainly in the tunica adventitia of the porcine aortic wall (Figure 2A) and the tunica adventitia of the human aortic wall (Figure 2B). To determine, whether the administered hGLuc mRNA could be translated into protein, the supernatants of ex vivo cultivated aortic vessels were collected at 24 and 48 h post-injection. Compared with control groups injected with Opti-MEM alone or Opti-MEM with L2000, microinjection of hGLuc mRNA in both human as well as porcine aortic vessels led to significantly increased luciferase activity after 24 h of incubation (Figure 2C). After 48 h of incubation, similar luciferase activity was measured in the supernatant of porcine vessels as after 24 h, indicating that most of the injected mRNA was translated in the first 24 h of incubation (Figure 2C, left). In human aortic vessels, slightly higher luciferase activity was detected after 48 h compared to 24 h. Although cell activity might be impaired in human pathogenic vessels derived from aortic aneurysms compared with healthy vessels, a significant amount of expressed luciferase protein was detected after 24 and 48 h of incubation after mRNA injection (Figure 2C, right).

The ability to microinject synthetic mRNA from the side of the adventitia was also tested in porcine aorta samples (data not shown). Here, the results were similar to those obtained with mRNA injections from the side of the intima, and after 24 and 48 h of incubation, increased luciferase activity was detected in aorta samples microinjected with hGLuc mRNA.

### 2.3 Production of TIMP-1 after the delivery of synthetic TIMP-1 mRNA in porcine aortic vessel wall

To determine, whether the administered TIMP-1 mRNA leads to increased TIMP-1 protein expression, the supernatants of ex vivo cultivated porcine aortic vessels were collected at 24 and 48 h post-injection with 3 µg TIMP-1 mRNA. Significantly increased TIMP-1 levels were detected after 48 h of incubation using ELISA (Figure 3).

### 2.4 Detection of MMP-9 activity after the application of synthetic TIMP-1 mRNA in porcine and human aortic vessel wall

After demonstrating that ex vivo aortic tissue can express the desired protein up to 48 h post-injection of synthetic mRNA using hGLuc encoding mRNA, the same ex vivo model was used to evaluate the functionality of expressed TIMP-1 protein. In situ zymography can visualize and assess the proteolytic activity of MMP-9 in histological sections using the fluorescently labeled MMP substrate DQ-gelatin. Enzymatic cleavage of DQ-gelatin by active MMPs results in a green fluorescence signal (Hadler-Olsen, E., et al., Gelatin in situ zymography on fixed, paraffin-embedded tissue: zinc and ethanol fixation preserve enzyme activity. J Histochem Cytochem, 2010. 58(1): p. 29-39). Since the activity of MMP-9 is inhibited by EDTA, which binds catalytically necessary Zn²⁺ ions, the tissue sections incubated with EDTA served as positive controls for the in situ zymography. TIMP-1 binds covalently to MMP-9 and inhibits DQ-gelatin cleavage. Thus, the ability of TIMP-1 protein, which is produced after the microinjection of TIMP-1 mRNA into the vessel wall, to inhibit MMP-9 was investigated in both porcine and human vessels. For this purpose, 5 µg of human synthetic TIMP-1 mRNA was injected ex vivo into porcine and human aortic vessels from the side of the intima and incubated for 24 or 48 h. After fixation, MMP-9 activity was assessed in paraffin sections by in situ zymography, MMP-9 dependent DQ-gelatin cleavage efficiency was determined, and TIMP-1 mRNA-treated versus untreated groups were compared. At both 24 and 48 h after injection of TIMP-1 mRNA from the side of the intima (Figure 4) or adventitia (Figure 7), a significantly lower signal of DQ-gelatin was detected in porcine vessel wall compared to the vessels treated with L2000 alone. As expected, the lowest MMP-9 activity was detected in EDTA-treated vessel sections.

Similar results were obtained in human aortic tissue after injection of TIMP-1 mRNA and incubation for 24 and 48 h (Figure 5). Here, a strongly reduced DQ-gelatin signal was observed both after 24 and 48 h after injection of synthetic TIMP-1 mRNA compared with L2000 control groups. Thereby, the inhibitory function of the produced TIMP-1 protein was confirmed. The proteolytic MMP-9 activity was successfully inhibited and decreased in mRNA-treated tissues.

### 2.5 Analysis of TIMP-1 mRNA translation efficiency by variation of nucleotide modifications

To improve the protein expression efficiency of the mRNA different nucleotide modifications were incorporated into the mRNA and tested. In the prior analysis, the nucleotide modifications Ψ/5mC were used. Ψ was replaced by me¹Ψ and TIMP-1 mRNA was modified with either me¹Ψ/5mC or me¹Ψ only. 1.5 µg of each mRNA variant was complexed with L2000 and 3×10⁵ NUFFs were transfected. After 24 h, protein translation efficiency was analyzed via TIMP-1 specific ELISA in supernatants (Figure 6). By the incorporation of me¹Ψ only, the protein expression efficiency was significantly enhanced. The additional modification with 5mC did not improve the translation.

### 3. Conclusion

The inventors provide an oligonucleotide allowing low-morbidity and high-efficiency treatment of cardiovascular diseases such as aneurysms and arteriosclerosis.

## Claims

1. An oligonucleotide comprising a nucleotide sequence encoding a 'tissue inhibitor of metalloproteases' (TIMP) protein for use in the treatment of diseases or medical conditions in a mammal.

2. The oligonucleotide for use of claim 1, **characterized in that** the disease or medical condition is associated with insufficient presence of at least one extracellular matrix protein, wherein the disease or medical condition is preferably selected from the group consisting of: aneurysm, fibrosis, arteriosclerosis, aortic stenosis, pulmonary emphysema, Williams-Beuren-Syndrome, subvalvular congenital aortic stenosis, myocardial infarction, stroke, rheumatoid arthritis, osteoarthritis, and tumour metastasis, more preferably selected from the group consisting of: aortic aneurysm, cranial aneurysm, peripheral aneurysm.

3. The oligonucleotide for use of any preceding claim, **characterized in that** the disease or medical condition is associated with an unphysiological ratio of MMP-to-TIMP activity, wherein preferably the unphysiological ratio of MMP-to-TIMP activity is associated with an enhanced MMP activity.

4. The oligonucleotide for use of any preceding claim, **characterized in that** the treatment is a prophylactic or therapeutic treatment, wherein preferably the treatment is selected from the group consisting of: prophylaxis of formation or rupture, and healing of aneurysms, said aneurysms are further preferably selected from the group consisting of: aortic aneurysm, cranial aneurysm, peripheral aneurysm.

5. The oligonucleotide for use of any preceding claim, **characterized in that** the oligonucleotide is an oligoribonucleotide, preferably an mRNA, wherein the oligoribonucleotide preferably comprises
- a 5'-Cap-structure, preferably a synthetic anti-reverse cap analogue, more preferably 3'-O-Me-m7G(5')ppp(5')G, and/or
- a polyA-tail, preferably consisting of at least approx. 70 adenine nucleotides, further preferably approx. 120 adenine nucleotides.

6. The oligonucleotide for use of any preceding claim, **characterized in that** at least one of the nucleotides is an analogue of a naturally occurring nucleotide, wherein the fraction of one type of naturally occurring nucleotide replaced by analogues is at least approx. 5 %, preferably at least approx. 25 %, more preferably approx. 100 %.

7. The oligonucleotide for use of claim 6, **characterized in that** the natural occurring nucleotides uridine and/or uridine are replaced by nucleotide analogues, preferably by a nucleotide analogue or a combination of analogues selected from the group consisting of: a combination of pseudouridine for replacement of uridine and 5-methylcytidine for replacement of cytidine, a combination of N¹-methylpseudouridine for replacement of uridine and 5-methylcytidine for replacement of cytidine, and N¹-methylpseudouridine for replacement of uridine, most preferably N¹-methylpseudouridine for replacement of uridine.

8. The oligonucleotide for use of any preceding claim, **characterized in that** the TIMP protein is a TIMP-1 protein, wherein the TIMP-1 protein preferably comprises the amino acid sequence of SEQ ID NO: 1.

9. The oligonucleotide for use of any preceding claim, comprising the nucleotide sequence of SEQ ID NO: 2.

10. The oligonucleotide for use of any preceding claim, **characterized in that** the oligonucleotide is complexed by polymers or lipids, preferably said oligonucleotide is contained within a lipid nanoparticle, or contained within a liposome.

11. A pharmaceutical composition, comprising the oligonucleotide for use of any of the preceding claims and a pharmaceutically acceptable carrier.

12. The pharmaceutical composition of claim 11, **characterized in that** the pharmaceutical composition is configured for local administration by injection into or external application onto the tissue of the mammal, wherein preferably the pharmaceutical composition is present in a formulation or delivery form selected from the group consisting of: a fluid, an implant, a balloon catheter, a vascular prosthesis, a microneedle, a patch, and a stent.

13. The pharmaceutical composition of claim 11, **characterized in that** the pharmaceutical composition is configured for systemic administration, wherein preferably the pharmaceutical composition is present in a formulation or delivery form selected from the group consisting of: a fluid or an implant.

14. The pharmaceutical composition of any of claims 11 to 13, **characterized in that** the pharmaceutical composition contains or is administered in combination with at least one antihypertensive drug.

15. A medical device, **characterized in that** the medical device comprises, includes, or is coated with the oligonucleotide for use of any of claims 1 to 10, wherein the medical device is preferably configured for permanent or transient presence in the body of the mammal, wherein further preferably the medical device is selected from the group consisting of: an implant, a balloon catheter, a vascular prosthesis, a microneedle, a patch, and a stent.
